# EUROPÄISCHE PATENTSCHRIFT

(11) **EP 3 402 769 B1**
(45) Veröffentlichungstag und Bekanntmachung des Hinweises auf die Patenterteilung: **25.03.2020**
(21) Anmeldenummer: 17700238.3
(22) Anmeldetag: 11.01.2017
(51) Int. Cl.: C07C 5/48, C07C 7/00, C07C 7/06, C07C 7/08, C07C 7/11, C07C 11/167

(54) **VERFAHREN ZUR HERSTELLUNG VON 1,3-BUTADIEN AUS N-BUTENEN DURCH OXIDATIVE DEHYDRIERUNG**
METHOD FOR PREPARING 1.3-BUTADIENE FROM N-BUTENES BY OXIDATIVE DEHYDRATION
PROCEDE DE FABRICATION DE 1,3-BUTADIENE A PARTIR DE N-BUTENES PAR DESHYDRATATION OXYDANTE

(30) Priorität: 13.01.2016 EP 16151045
(43) Veröffentlichungstag der Anmeldung: 21.11.2018
(73) Patentinhaber: BASF SE, 67056 Ludwigshafen am Rhein (DE); Linde AG, 80331 München (DE)
(72) Erfinder: JOSCH, Jan Pablo, 67056 Ludwigshafen (DE); KARANIKOULIS, Georgios, 67056 Ludwigshafen (DE); HAMMEN, Oliver, 67056 Ludwigshafen (DE); MOSSBACHER, Claudia, 67056 Ludwigshafen (DE); WENNING, Ulrike, 80331 München (DE); WELLENHOFER, Anton, 80331 München (DE); TOEGEL, Christine, 80331 München (DE); REYNEKE, Hendrik, 80331 München (DE)
(74) Vertreter: Schuck, Alexander
(86) Internationale Anmeldenummer: PCT/EP2017/050438
(87) Internationale Veröffentlichungsnummer: WO 2017/121739

(56) Entgegenhaltungen:
- WO-A1-2014/111409
- DATABASE WPI Week 201107 Thomson Scientific, London, GB; AN 2011-A58751 XP002759141, & JP 2011 006381 A (MITSUBISHI CHEM CORP) 13. Januar 2011 (2011-01-13) in der Anmeldung erwähnt

## Beschreibung

Die Erfindung betrifft ein Verfahren zur Herstellung von 1,3-Butadien aus n-Butenen durch oxidative Dehydrierung (ODH), bei dem im Aufarbeitungsteil ein Polymerisationsinhibitor zugegeben wird.

Butadien ist eine bedeutende Grundchemikalie und wird beispielsweise zur Herstellung von Synthesekautschuken (Butadien-Homopolymere, Styrol-Butadien-Kautschuk oder NitrilKautschuk) oder zur Herstellung von thermoplastischen Terpolymeren (Acrylnitril-Butadien-Styrol-Copolymere) eingesetzt. Butadien wird ferner zu Sulfolan, Chloropren und 1,4-Hexamethylendiamin (über 1,4-Dichlorbuten und Adipinsäuredinitril) umgesetzt. Durch Dimerisierung von Butadien kann ferner Vinylcyclohexen erzeugt werden, welches zu Styrol dehydriert werden kann.

Butadien kann durch thermische Spaltung (Steam-Cracken) gesättigter Kohlenwasserstoffe hergestellt werden, wobei üblicherweise von Naphtha als Rohstoff ausgegangen wird. Beim Steam-Cracken von Naphtha fällt ein Kohlenwasserstoff-Gemisch aus Methan, Ethan, Ethen, Acetylen, Propan, Propen, Propin, Allen, Butanen, Butenen, Butadien, Butinen, Methylallen, C₅- und höheren Kohlenwasserstoffen an.

Butadien kann auch durch oxidative Dehydrierung von n-Butenen (1-Buten und/oder 2-Buten) erhalten werden. Als Ausgangsgasgemisch für die oxidative Dehydrierung (Oxidehydrierung, ODH) von n-Butenen zu Butadien kann jedes beliebige n-Butene enthaltende Gemisch eingesetzt werden. Beispielsweise kann eine Fraktion verwendet werden, die als Hauptbestandteil n-Butene (1-Buten und/oder 2-Buten) enthält und aus der C₄-Fraktion eines Naphtha-Crackers durch Abtrennen von Butadien und Isobuten erhalten wurde. Des Weiteren können auch Gasgemische als Ausgangsgas eingesetzt werden, die 1-Buten, cis-2-Buten, trans-2-Buten oder deren Gemische umfassen und durch Dimerisierung von Ethylen erhalten wurden. Ferner können als Ausgangsgas n-Butene enthaltende Gasgemische eingesetzt werden, die durch katalytisches Wirbelschichtcracken (Fluid Catalytic Cracking, FCC) erhalten wurden.

Verfahren zur oxidativen Dehydrierung von Butenen zu Butadien sind grundsätzlich bekannt. Ein derartiges Verfahren wird zum Beispiel in WO2014111409 beschrieben und umfasst die nachstehenden Schritte:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b durch Inkontaktbringen mit einem Kühlmittel und Kondensation zumindest eines Teils der hochsiedenden Nebenkomponenten;
Cb) Kompression des verbleibenden Produktgasstroms b in mindestens einer Kompressionsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
Da) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein C₄-Produktgasstrom d1 erhalten wird.

US 2012/0130137A1 beispielsweise beschreibt ein Verfahren zur oxidativen Dehydrierung von Butenen zu Butadien unter Verwendung von Katalysatoren, die Oxide von Molybdän, Bismuth und in der Regel weiteren Metallen enthalten.

In Absatz [0122] wird auch auf die Problematik der Nebenprodukte hingewiesen. Insbesondere werden Phthalsäureanhydrid, Anthrachinon und Fluorenon genannt, die typischerweise in Konzentrationen von 0,001 bis 0,10 Vol.-% im Produktgas vorlägen. In der US 2012/0130137A1 Absatz [0124] bis [0126] wird empfohlen, die heißen Reaktoraustragsgase direkt durch Kontakt mit einer Kühlflüssigkeit (Quench-Turm) auf zunächst 5 bis 100 °C abzukühlen. Als Kühlflüssigkeiten werden Wasser oder wässrige Alkali-Lösungen genannt. Ausdrücklich wird die Problematik von Verstopfungen im Quench durch Hochsieder aus dem Produktgas oder durch Polymerisationsprodukte hochsiedender Nebenprodukte aus dem Produktgas erwähnt, weshalb es vorteilhaft sei, dass hochsiedende Nebenprodukte so wenig wie möglich aus dem Reaktionsteil in den Kühlungsteil (Quench) ausgetragen werden. Eine Abtrennung von iso-Buten, von dessen Umsetzungsprodukt Methacrolein, von Acetaldehyd oder von Acrolein wird nicht erwähnt.

In JP 2011-001341A wird für ein Verfahren zur oxidativen Dehydrierung von Alkenen zu konju-gierten Alkadienen eine zweistufige Kühlung beschrieben. Dabei wird das Produktaustragsgas der oxidativen Dehydrierung zunächst auf eine Temperatur zwischen 300 und 221 °C eingestellt und dann auf eine Temperatur zwischen 99 und 21 °C weiter abgekühlt. In den Absätzen [0066] ff. wird beschrieben, dass zur Einstellung der Temperatur zwischen 300 und 221 °C bevorzugt Wärmetauscher eingesetzt werden, wobei aber auch ein Teil der Hochsieder aus dem Produkt-gas in diesen Wärmetauschern ausfallen könnten. In der JP2011-001341A wird deshalb ein gelegentliches Auswaschen von Ablagerungen aus den Wärmetauschern mit organischen oder wässrigen Lösungsmitteln beschrieben. Als Lösungsmittel werden beispielsweise aromatische Kohlenwasserstoffe wie Toluol oder Xylol oder ein alkalisches wässriges Lösungsmittel wie beispielsweise die wässrige Lösung von Natriumhydroxid beschrieben. Um ein zu häufiges Abstellen des Verfahrens zur Reinigung des Wärmetauscher zu vermeiden, wird in der JP 2011-001341A ein Aufbau mit zwei parallel angeordneten Wärmetauschern beschrieben, die jeweils abwechselnd betrieben oder gespült werden (sogenannte A/B-Fahrweise). Eine Abtrennung von iso-Buten oder von dessen Umsetzungsprodukt Methacrolein oder von Acetaldehyd und Acrolein wird nicht erwähnt.

JP 2011-132218 begrenzt den iso-Buten-Gehalt im Feed, da bekannt ist, dass iso-Buten Oxygenate bildet. Die Abtrennung der Oxygenate wird aber nicht beschrieben.

JP 2012240963 beschreibt ein Verfahren zur Butadienherstellung, in dem der C₄-Kohlenwasserstoffe enthaltene Gasstrom in einer Absorbtionsstufe b' mit einem Absorbens b in Kontakt gebracht wird, um die C₄-Komponenten zu absorbieren.

JP 2010-090083 begrenzt die Menge an Aldehyden und offenbart in Tabelle 1 auch die Bildung von Methacrolein, macht aber keine Vorschläge zu dessen Abtrennung.

Iso-Buten ist in nahezu allen C₄-Kohlenwasserstoffströmen, die für den ODH-Prozess Verwendung finden können, enthalten. Insbesondere C₄-Kohlenwasserstoffströme aus FC-Crackern enthalten iso-Buten in Mengen von bis zu 15 Vol.-%. Das in den ODH-Reaktor eintretende iso-Buten wird, abhängig vom verwendeten Katalysator und den Reaktionsbedingungen, zu ca. 50% zu Methacrolein umgesetzt. Dieses reichert sich im Kreislaufstrom des Absorptions-/Desorptionsteil der C₄-Kohlenwasserstoff-Abtrennung an und kann Nebenreaktionen wie Oligomerisierungen und Polymerisationen, Ablagerungen auf den Kolonneneinbauten und insbesondere an Verdampfern und Kondensatoren sowie eine Verschlechterung der Trennleistung verursachen.

Es wurde gefunden, dass die im Produktgasstrom des ODH-Reaktors enthaltenen Nebenkomponenten in Bereichen der nachgeschalteten Aufarbeitungsstufen, in denen hohe Konzentrationen an C₄-Kohlenwasserstoffen vorliegen, die Polymerbildung initiieren und fördern.

In JP 2011-006381 A wird das Risiko von Peroxidbildung im Aufarbeitungsteil eines Verfahrens zur Herstellung von konjugierten Alkadienen angesprochen. Zur Lösung dieses Problems wird der Zusatz von Polymerisationsinhibitoren zu den Absorptionslösungen der C₄-Kohlenwasserstoffabtrennung und die Einstellung eines maximalen Peroxidgehalts von 100 Gewichts-ppm durch Erhitzen der Absorptionslösungen beschrieben.

Aufgabe der Erfindung ist es, ein verbessertes Verfahren zur Herstellung von Butadien durch oxidative Deyhdrierung von n-Butenen und anschließender Aufarbeitung des C₄-Kohlenwasser-stoffe und Nebenkomponenten enthaltenden Produktgasstroms bereitzustellen, das den oben beschriebenen Nachteilen abhilft. Insbesondere soll ein Verfahren bereitgestellt werden, bei dem die Polymerbildung bei der Abtrennung der C₄-Kohlenwasserstoffe aus dem Produktgasstrom des ODH-Reaktors im Aufarbeitungsteil des Verfahrens minimiert wird.

Gelöst wird die Aufgabe durch ein Verfahren zur Herstellung von Butadien aus n-Butenen mit den Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b durch Inkontaktbringen mit einem Kühlmittel und Kondensation zumindest eines Teils der hochsiedenden Nebenkomponenten;
Cb) Kompression des verbleibenden Produktgasstroms b in mindestens einer Kompressionsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
Da) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein C₄-Produktgasstrom d1 erhalten wird,
dadurch gekennzeichnet, dass in Schritt Db) am Kolonnenkopf der Desorptionskolonne ein Polymerisationsinhibitor zugegeben wird.

Im Allgemeinen befindet sich am Kolonnenkopf der Desorptionskolonne ein Kopfkondensator. Vorzugsweise wird der Polymerisationsinhibitor im Bereich des Kopfkondensators zugegeben.

Es wurde gefunden, dass die Polymerbildung in der Desorptionskolonne, im Kopfkondensator der Desorptionskolonne, im Kopfkondensator-Kreislauf sowie in nachgeschalteten Verdampfern durch die Zugabe von Polymerisationsinhibitoren minimiert werden kann.

Vorzugsweise wird der Polymerisationsinhibitor in solchen Mengen zugegeben, dass die Konzentration des Polymerisationsinhibitors in dem am Kopfkondensator erhaltenen flüssigen Kondensatstrom von 10 bis 500 ppm beträgt.

Vorzugsweise werden noch die Schritte E) und F) durchgeführt:
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

Im Allgemeinen wird in der Abkühlstufe Ca) ein wässriges Kühlmittel oder ein organisches Lösungsmittel verwendet.

Vorzugsweise wird in der Abkühlstufe Ca) ein organisches Lösungsmittel verwendet. Diese weisen im Allgemeinen ein sehr viel höheres Lösungsvermögen für die hochsiedenden Nebenprodukte, die in den dem ODH-Reaktor nachgelagerten Anlageteilen zu Ablagerungen und Verstopfungen führen können, als Wasser oder alkalisch-wässrige Lösungen auf. Bevorzugte als Kühlmittel eingesetzte organische Lösungsmittel sind aromatische Kohlenwasserstoffe, beispielsweise Toluol, o-Xylol, m-Xylol, p-Xylol, Diethylbenzole, Trietylbenzole, Diisopropylbenzole, Triisopropylbenzole und Mesitylen (TMB) oder Gemische daraus. Besonders bevorzugt ist Mesitylen.

Nachstehende Ausführungsformen sind bevorzugte oder besonders bevorzugte Varianten des erfindungsgemäßen Verfahrens.

Die Stufe Ca) wird mehrstufig in Stufen Ca1) bis Can), bevorzugt zweistufig in zwei Stufen Ca1) und Ca2) durchgeführt. Dabei wird besonders bevorzugt zumindest ein Teil des Lösungsmittels nach Durchlaufen der zweiten Stufe Ca2) als Abkühlmittel der ersten Stufe Ca1) zugeführt.

Die Stufe Cb) umfasst im Allgemeinen mindestens eine Kompressionsstufe Cba) und mindestens eine Abkühlstufe Cbb). Bevorzugt wird mindestens eine Abkühlstufe Cbb), in der das in der Kompressionsstufe Cba) komprimierte Gas mit einem Abkühlmittel in Kontakt gebracht wird. Besonders bevorzugt enthält das Abkühlmittel der Abkühlstufe Cbb) das gleiche organische Lösungsmittel, das in der Stufe Ca) als Abkühlmittel verwendet wird. In einer besonders bevorzugten Variante wird zumindest ein Teil dieses Abkühlmittels nach Durchlaufen der mindestens einen Abkühlstufe Cbb) als Abkühlmittel der Stufe Ca) zugeführt.

Bevorzugt umfasst die Stufe Cb) mehrere Kompressionsstufen Cba1) bis Cban) und Abkühlstufen Cbb1) bis Cbbn), beispielsweise vier Kompressionsstufen Cba1) bis Cba4) und vier Abkühlstufen Cbb1) bis Cbb4).

Bevorzugt umfasst Schritt D) die Schritte Da1), Da2) und Db):
Da1) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Da2) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Db) Desorption der C₄-Kohlenwasserstoffe aus dem beladenen Absorptionsmittelstrom, wobei ein C₄-Produktgasstrom d1 erhalten wird, der im Wesentlichen aus C₄-Kohlenwasserstoffen besteht und weniger als 100 ppm Sauerstoff umfasst.

Bevorzugt ist das in Schritt Da) eingesetzte hochsiedende Absorptionsmittel ein aromatisches Kohlenwasserstofflösungsmittel, besonders bevorzugt es das in Schritt Ca) eingesetzte aromatische Kohlenwasserstofflösungsmittel, insbesondere Mesitylen. Verwendet werden können auch Diethylbenzole, Trietylbenzole, Diisopropylbenzole und Triisopropy-Ibenzole.

In einer Ausführungsform der Erfindung wird der in Schritt Da) enthaltene Gasstrom d2 zu mindestens 30%, bevorzugt zu mindestens 40% in den Schritt B) zurückgeführt. Das kann dann sinnvoll sein, wenn nur ein geringer Purgestrom aus dem Gasstrom d2 ausgeschleust werden muss.

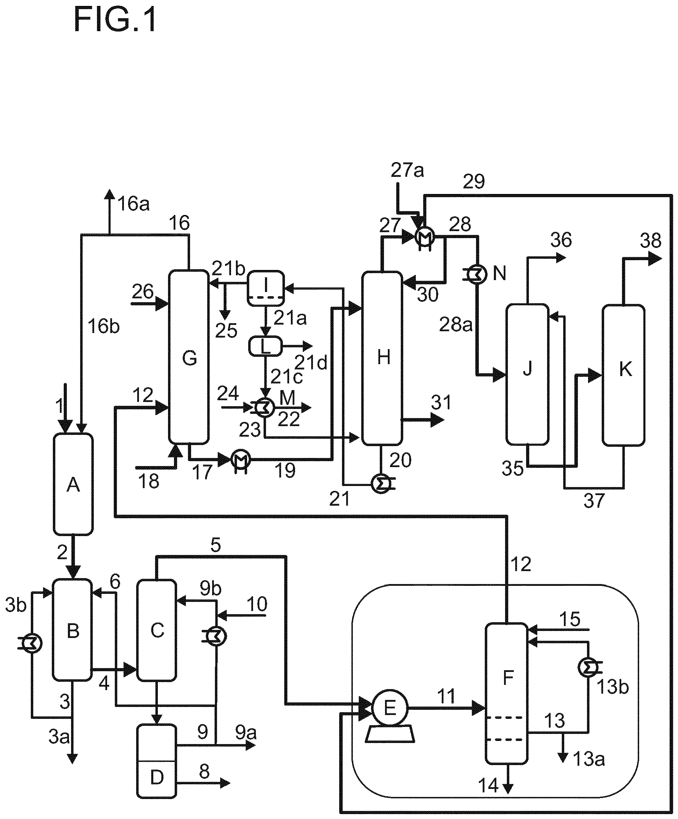

Eine Ausführungsform des erfindungsgemäßen Verfahrens ist in Figur 1 dargestellt und wird im Folgenden detailliert beschrieben.

Als Einsatzgasstrom werden n-Butene (1-Buten und/oder cis-/trans-2-Buten) und iso-Buten enthaltende Gasgemische eingesetzt. Ein solches Gasgemisch kann beispielsweise durch nicht-oxidative Dehydrierung von n-Butan erhalten werden. Es kann auch eine Fraktion eingesetzt werden, die als Hauptbestandteil n-Butene (1-Buten und cis-/trans-2-Buten) enthält und aus der C₄-Fraktion des Naphtha-Crackers durch Abtrennung von Butadien und iso-Buten erhalten wurde. Des Weiteren können auch Gasgemische als Eingangsgasstrom eingesetzt werden, die 1-Buten, cis-2-Buten, trans-2-Buten oder Mischungen daraus umfassen, und die durch Dimerisierung von Ethylen erhalten wurden. Ferner können als Eingangsgasstrom n-Butene enthaltende Gasgemische eingesetzt werden, die durch katalytisches Wirbelschichtcracken (Fluid Catalytic Cracking, FCC) erhalten wurden.

In einer Ausführungsform des erfindungsgemäßen Verfahrens wird das n-Butene enthaltende Ausgangsgasgemisch durch nicht-oxidative Dehydrierung von n-Butan erhalten. Durch die Kopplung einer nicht-oxidativen katalytischen Dehydrierung mit der oxidativen Dehydrierung der gebildeten n-Butene kann eine hohe Ausbeute an Butadien, bezogen auf eingesetztes n-Butan, erhalten werden. Bei der nicht-oxidativen katalytischen n-Butan-Dehydrierung wird ein Gasgemisch erhalten, das neben Butadien 1-Buten, 2-Buten und nicht umgesetztem n-Butan Nebenbestandteile enthält. Übliche Nebenbestandteile sind Wasserstoff, Wasserdampf, Stickstoff, CO und CO₂, Methan, Ethan, Ethen, Propan und Propen. Die Zusammensetzung des die erste Dehydrierzone verlassenden Gasgemischs kann abhängig von der Fahrweise der Dehydrierung stark variieren. So weist bei Durchführung der Dehydrierung unter Einspeisung von Sauerstoff und zusätzlichem Wasserstoff das Produktgasgemisch einen vergleichsweise hohen Gehalt an Wasserdampf und Kohlenstoffoxiden auf. Bei Fahrweisen ohne Einspeisung von Sauerstoff weist das Produktgasgemisch der nicht-oxidativen Dehydrierung einen vergleichsweise hohen Gehalt an Wasserstoff auf.

In Schritt B) werden der n-Butene enthaltende Einsatzgasstrom und ein sauerstoffhaltiges Gas in mindestens eine Dehydrierzone (den ODH-Reaktor A) eingespeist und die in dem Gasgemisch enthaltenen Butene in Gegenwart eines Oxidehydrierungskatalysators oxidativ zu Butadien dehydriert.

In einer Ausführungsform ist bevorzugt, ein sauerstoffhaltiges Gas zu verwenden, das mehr als 10 Vol.-%, vorzugsweise mehr als 15 Vol.-% und besonders bevorzugt mehr als 20 Vol.-% molekularen Sauerstoff enthält. In einer Ausführungsform wird Luft als sauerstoffhaltiges Gas eingesetzt. Die Obergrenze für den Gehalt an molekularem Sauerstoff in dem sauerstoffhaltigen Gas beträgt dann im Allgemeinen 50 Vol.-% oder weniger, vorzugsweise 30 Vol.-% oder weniger und noch mehr bevorzugt 25 Vol.-% oder weniger. Darüber hinaus können in dem molekularen Sauerstoff enthaltenden Gas beliebige Inertgase enthalten sein. Als mögliche Inertgase können Stickstoff, Argon, Neon, Helium, CO, CO₂ und Wasser genannt werden. Die Menge an Inertgasen in dem sauerstoffhaltigen Gas beträgt für Stickstoff im Allgemeinen 90 Vol.-% oder weniger, vorzugsweise 85 Vol.-% oder weniger und noch mehr bevorzugt 80 Vol.-% oder weniger. Im Falle anderer Bestandteile als Stickstoff in dem sauerstoffhaltigen Gas beträgt sie im Allgemeinen 10 Vol.-% oder weniger, vorzugsweise 1 Vol.-% oder weniger.

Für die Oxidehydrierung geeignete Katalysatoren basieren im Allgemeinen auf einem Mo-Bi-O-haltigen Multimetalloxidsystem, das in der Regel zusätzlich Eisen enthält. Im Allgemeinen enthält das Katalysatorsystem noch weitere zusätzliche Komponenten, wie beispielsweise Kalium, Cäsium, Magnesium, Zirkon, Chrom, Nickel, Cobalt, Cadmium, Zinn, Blei, Germanium, Lanthan, Mangan, Wolfram, Phosphor, Cer, Aluminium oder Silizium. Auch eisenhaltige Ferrite wurden als Katalysatoren vorgeschlagen.

In einer bevorzugten Ausführungsform enthält das Multimetalloxid Cobalt und/oder Nickel. In einer weiteren bevorzugten Ausführungsform enthält das Multimetalloxid Chrom. In einer weiteren bevorzugten Ausführungsform enthält das Multimetalloxid Mangan.

Beispiele für Mo-Bi-Fe-O-haltige Multimetalloxide sind Mo-Bi-Fe-Cr-O- oder Mo-Bi-Fe-Zr-O-haltige Multimetalloxide. Bevorzugte Systeme sind beispielsweise beschrieben in US 4,547,615 (Mo₁₂BiFe_{0,1}Ni₈ZrCr₃K_{0,2}Oₓ und Mo₁₂BiFe_{0,1}Ni₈AlCr₃K_{0,2}Oₓ), US 4,424,141 (Mo₁₂BiFe₃Co_{4,5}Ni_{2,5}P_{0,5}K_{0,1}Oₓ + SiO₂), DE-A25 30 959 (Mo₁₂BiFe₃Co_{4,5}Ni_{2,5}Cr_{0,5}K_{0,1}Oₓ, Mo_{13,75}BiFe₃Co_{4,5}Ni_{2,5}Ge_{0,5}K_{0,8}Oₓ, Mo₁₂BiFe₃Co_{4,5}Ni_{2,5}Mn_{0,5}K_{0,5}Oₓ und Mo₁₂BiFe₃Co_{4,5}Ni_{2,5}La_{0,5}K_{0,1}Oₓ), US 3,911,039 (Mo₁₂BiFe₃Co_{4,5}Ni_{2,5}Sn_{0,5}K_{0,1}Oₓ), DE-A 25 30 959 und DE-A 24 47 825 (Mo₁₂BiFe₃Co_{4,5}Ni_{2,5}W_{0,5}K_{0,1}Oₓ).

Geeignete Multimetalloxide und deren Herstellung sind weiterhin beschrieben in US 4,423,281 (Mo₁₂BiNi₈Pb_{0,5}Cr₃K_{0,2}Oₓ und Mo₁₂Bi_{b}Ni₇Al₃Cr_{0,5}K_{0,5}Oₓ), US 4,336,409 (Mo₁₂BiNi₆Cd₂Cr₃P_{0,5}Oₓ), DE-A 26 00 128 (Mo₁₂BiNi_{0,5}Cr₃P_{0,5}Mg_{7,5}K_{0,1}Oₓ + SiO₂) und DE-A 24 40 329 (Mo₁₂BiCo_{4,5}Ni_{2,5}Cr₃P_{0,5}K_{0,1}Oₓ).

Besonders bevorzugte katalytisch aktive, Molybdän und mindestens ein weiteres Metall enthaltende Multimetalloxide weisen die allgemeine Formel (la) auf:

Mo₁₂BiₐFe_{b}Co_{c}Ni_{d}CrₑX¹_{f}X²_{g}O_{y} (Ia),

mit
X¹ = Si, Mn und/oder Al,
X² = Li, Na, K, Cs und/oder Rb,
0,2 ≤ a ≤ 1,
0,5 ≤ b ≤ 10,
0 ≤ c ≤ 10,
0 ≤ d ≤ 10,
2 ≤ c+d ≤ 10,
0 ≤ e ≤ 2,
0 ≤ f ≤ 10,
0 ≤ g ≤ 0,5,
y = eine Zahl, die unter der Voraussetzung der Ladungsneutralität durch die Wertigkeit und Häufigkeit der von Sauerstoff verschiedenen Elemente in (la) bestimmt wird.

Bevorzugt sind Katalysatoren, deren katalytisch aktive Oxidmasse von den beiden Metallen Co und Ni nur Co aufweist (d = 0). Bevorzugte ist X¹ Si und/oder Mn und X² ist vorzugsweise K, Na und/oder Cs, besonders bevorzugt ist X² = K. Besonders bevorzugt ist ein weitgehend Cr(VI)-freier Katalysator.

Zur Durchführung der oxidativen Dehydrierung bei hohem Gesamtumsatz von n-Butenen ist ein Gasgemisch bevorzugt, welches ein molares Sauerstoff : n-Butene-Verhältnis von mindestens 0,5 aufweist. Bevorzugt wird bei einem Sauerstoff : n-Butene-Verhältnis von 0,55 bis 10 gearbeitet. Zur Einstellung dieses Wertes kann das Ausgangsstoffgas mit Sauerstoff oder einem sauerstoffhaltigem Gas und gegebenenfalls zusätzlichem Inertgas, Methan oder Wasserdampf vermischt werden. Das erhaltene sauerstoffhaltige Gasgemisch wird dann der Oxidehydrierung zugeführt.

Die Reaktionstemperatur der Oxidehydrierung wird im Allgemeinen durch ein Wärmeaustauschmittel, welches sich um die Reaktionsrohre herum befindet, kontrolliert. Als solche flüssige Wärmeaustauschmittel kommen z. B. Schmelzen von Salzen oder Salzgemischen wie Kaliumnitrat, Kaliumnitrit, Natriumnitrit und/oder Natriumnitrat sowie Schmelzen von Metallen wie Natrium, Quecksilber und Legierungen verschiedener Metalle in Betracht. Aber auch ionische Flüssigkeiten oder Wärmeträgeröle sind einsetzbar. Die Temperatur des Wärmeaustauschmittels liegt zwischen 220 bis 490 °C und bevorzugt zwischen 300 bis 450 °C und besonders bevorzugt zwischen 350 und 420 °C.

Aufgrund der Exothermie der ablaufenden Reaktionen kann die Temperatur in bestimmten Abschnitten des Reaktorinneren während der Reaktion höher liegen als diejenige des Wärmeaustauschmittels, und es bildet sich ein sogenannter Hotspot aus. Die Lage und Höhe des Hotspots ist durch die Reaktionsbedingungen festgelegt, aber sie kann auch durch das Verdünnungsverhältnis der Katalysatorschicht oder den Durchfluss an Mischgas reguliert werden. Die Differenz zwischen Hotspot-Temperatur und der Temperatur des Wärmeaustauschmittels liegt im Allgemeinen zwischen 1 bis 150 °C, bevorzugt zwischen 10 bis 100 °C und besonders bevorzugt zwischen 20 bis 80 °C. Die Temperatur am Ende des Katalysatorbettes liegt im Allgemeinen zwischen 0 bis 100 °C, vorzugsweise zwischen 0,1 bis 50 °C, besonders bevorzugt zwischen 1 bis 25 °C oberhalb der Temperatur des Wärmeaustauschmittels.

Die Oxidehydrierung kann in allen aus dem Stand der Technik bekannten Festbettreaktoren durchgeführt werden, wie beispielsweise im Hordenofen, im Festbettrohr- oder Rohrbündelreaktor oder im Plattenwärmetauscherreaktor. Ein Rohrbündelreaktor ist bevorzugt.

Vorzugsweise wird die oxidative Dehydrierung in Festbettrohrreaktoren oder Festbettrohrbündelreaktoren durchgeführt. Die Reaktionsrohre werden (ebenso wie die anderen Elemente des Rohrbündelreaktors) in der Regel aus Stahl gefertigt. Die Wanddicke der Reaktionsrohre beträgt typischerweise 1 bis 3 mm. Ihr Innendurchmesser liegt in der Regel (einheitlich) bei 10 bis 50 mm oder bei 15 bis 40 mm, häufig bei 20 bis 30 mm. Die im Rohrbündelreaktor untergebrachte Anzahl an Reaktionsrohren beläuft sich in der Regel wenigstens auf 1000, oder 3000, oder 5000, vorzugsweise auf wenigstens 10 000. Häufig beträgt die Anzahl der im Rohrbündelreaktor untergebrachten Reaktionsrohre 15 000 bis 30 000 bzw. bis 40 000 oder bis 50 000. Die Länge der Reaktionsrohre erstreckt sich im Normalfall auf wenige Meter, typisch ist eine Reaktionsrohrlänge im Bereich von 1 bis 8 m, häufig 2 bis 7 m, vielfach 2,5 bis 6 m.

Weiterhin kann die Katalysatorschicht, die im ODH-Reaktor A eingerichtet ist, aus einer einzelnen Schicht oder aus 2 oder mehr Schichten bestehen. Diese Schichten können aus reinem Katalysator bestehen oder mit einem Material verdünnt sein, das nicht mit dem Ausgangsgas oder Komponenten aus dem Produktgas der Reaktion reagiert. Weiterhin können die Katalysatorschichten aus Vollmaterial und/oder geträgerten Schalenkatalysatoren bestehen.

Der die oxidative Dehydrierung verlassende Produktgasstrom 2 enthält neben Butadien im Allgemeinen noch nicht umgesetztes 1-Buten und 2-Buten, Sauerstoff sowie Wasserdampf. Als Nebenkomponenten enthält er weiterhin im Allgemeinen Kohlenmonoxid, Kohlendioxid, Inertgase (hauptsächlich Stickstoff), leichtsiedende Kohlenwasserstoffe wie Methan, Ethan, Ethen, Propan und Propen, Butan und iso-Butan, gegebenenfalls Wasserstoff sowie gegebenenfalls sauerstoffhaltige Kohlenwasserstoffe, sogenannte Oxygenate.

Oxygenate können beispielsweise Formaldehyd, Furan, Essigsäure, Maleinsäureanhydrid, Ameisensäure, Methacrolein, Methacrylsäure, Crotonaldehyd, Crotonsäure, Propionsäure, Acrylsäure, Methylvinylketon, Styrol, Benzaldehyd, Benzoesäure, Phthalsäureanhydrid, Fluorenon, Anthrachinon und Butyraldehyd sein.

Der Produktgasstrom 2 am Reaktorausgang ist durch eine Temperatur nahe der Temperatur am Ende des Katalysatorbetts charakterisiert. Der Produktgasstrom wird dann auf eine Temperatur von 150 bis 400 °C, bevorzugt 160 bis 300 °C, besonders bevorzugt 170 bis 250 °C gebracht. Es ist möglich, die Leitung, durch die der Produktgasstrom fließt, um die Temperatur im gewünschten Bereich zu halten, zu isolieren oder einen Wärmetauscher einzusetzen. Dieses Wärmetauschersystem ist beliebig, solange mit diesem System die Temperatur des Produktgases auf dem gewünschten Niveau gehalten werden kann. Als Beispiel eines Wärmetauschers können Spiralwärmetauscher, Plattenwärmetauscher, Doppelrohrwärmetauscher, Multirohrwärmetauscher, Kessel-Spiralwärmetauscher, Kessel-Mantelwärmetauscher, Flüssigkeit-Flüssigkeit-Kontakt-Wärmetauscher, Luft-Wärmetauscher, Direktkontaktwärmetauscher sowie Rippenrohrwärmetauscher genannt werden. Da, während die Temperatur des Produktgases auf die gewünschte Temperatur eingestellt wird, ein Teil der hochsiedenden Nebenprodukte, die im Produktgas enthalten sind, ausfallen kann, sollte daher das Wärmetauschersystem vorzugsweise zwei oder mehr Wärmetauscher aufweisen. Falls dabei zwei oder mehr vorgesehene Wärmetauscher parallel angeordnet sind, und so eine verteilte Kühlung des gewonnenen Produktgases in den Wärmetauschern ermöglicht wird, nimmt die Menge an hochsiedenden Nebenprodukten, die sich in den Wärmetauschern ablagern, ab und so kann ihre Betriebsdauer verlängert werden. Als Alternative zu der oben genannten Methode können die zwei oder mehr vorgesehenen Wärmetauscher parallel angeordnet sein. Das Produktgas wird einem oder mehreren, nicht aber allen, Wärmetauschern zugeführt, welche nach einer gewissen Betriebsdauer von anderen Wärmetauschern abgelöst werden. Bei dieser Methode kann die Kühlung fortgesetzt werden, ein Teil der Reaktionswärme zurückgewonnen und parallel dazu können die in einem der Wärmetauscher abgelagerten hochsiedenden Nebenprodukte entfernt werden. Als ein oben genanntes Kühlmittel kann ein Lösungsmittel, solange es in der Lage ist, die hochsiedenden Nebenprodukte aufzulösen, verwendet werden. Beispiele sind aromatische Kohlenwasserstofflösungsmittel, wie z.B. Toluol, Xylole, Diethylbenzole, Trietylbenzole, Diisopropylbenzole und Triisopropylbenzole. Besonders bevorzugt ist Mesitylen. Es können auch wässrige Lösungsmittel verwendet werden. Diese können sowohl sauer als auch alkalisch gestellt werden, wie zum Beispiel eine wässrige Lösung von Natriumhydroxid.

Anschließend wird aus dem Produktgasstrom 2 durch Abkühlung und Kompression ein Großteil der hochsiedenden Nebenkomponenten und des Wassers abgetrennt werden. Die Abkühlung erfolgt durch Inkontaktbringen mit einem Kühlmittel. Diese Stufe wird nachfolgend auch als Quench bezeichnet. Dieser Quench kann aus nur einer Stufe oder aus mehreren Stufen bestehen (beispielsweise B, C in Figur 1). Der Produktgasstrom 2 wird also direkt mit dem organischen Kühlmedium 3b und 9b in Kontakt gebracht und dadurch gekühlt. Als Kühlmedium geeignet sind wässrige Kühlmittel oder organische Lösungsmittel, bevorzugt aromatische Kohlenwasserstoffe, besonders bevorzugt Toluol, o-Xylol, m-Xylol, p-Xylol oder Mesitylen, oder Gemische daraus. Verwendet werden können auch Diethylbenzol, Trietylbenzol, Diisopropylbenzol und Triisopropylbenzol.

Bevorzugt ist ein zweistufiger Quench (umfassend die Stufen B und C gemäß Figur 1), d.h. die Stufe Ca) umfasst zwei Abkühlstufen Ca1) und Ca2), in denen der Produktgasstrom 2 mit dem organischen Lösungsmittel in Kontakt gebracht wird.

Im Allgemeinen hat das Produktgas 2, je nach Vorliegen und Temperaturniveau eines Wärmetauschers vor dem Quench B, eine Temperatur von 100 bis 440 °C. Das Produktgas wird in der 1. Quenchstufe B mit dem Kühlmedium aus organischem Lösungsmittel in Kontakt gebracht. Hierbei kann das Kühlmedium durch eine Düse eingebracht werden, um eine möglichst effiziente Durchmischung mit dem Produktgas zu erreichen. Zum gleichen Zweck können in der Quenchstufe Einbauten, wie zum Beispiel weitere Düsen, eingebracht werden, die das Produktgas und das Kühlmedium gemeinsam passieren. Der Kühlmitteleinlass in den Quench ist so ausgelegt, dass ein Verstopfen durch Ablagerungen im Bereich des Kühlmitteleinlasses minimiert wird.

Im Allgemeinen wird das Produktgas 2 in der ersten Quenchstufe B auf 5 bis 180 °C, vorzugsweise auf 30 bis 130 °C und noch mehr bevorzugt auf 60 bis 110 °C gekühlt. Die Temperatur des Kühlmittelmediums 3b am Einlass kann im Allgemeinen 25 bis 200 °C, bevorzugt 40 bis 120 °C, insbesondere bevorzugt 50 bis 90 °C betragen. Der Druck in der ersten Quenchstufe B ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Wenn größere Mengen hochsiedende Nebenprodukte im Produktgas vorhanden sind, kann es leicht zur Polymerisation von hochsiedenden Nebenprodukten und zu Ablagerungen von Feststoffen, die durch hochsiedende Nebenprodukte in diesem Verfahrensabschnitt verursacht werden, kommen. Im Allgemeinen ist die Quenchstufe B als Kühlturm ausgestaltet. Das im Kühlturm eingesetzte Kühlmedium 3b wird häufig zirkulierend eingesetzt. Der Kreislaufstrom des Kühlmediums in Liter pro Stunde, bezogen auf den Massenstrom an Butadien in Gramm pro Stunde, kann im Allgemeinen 0,0001 bis 5 l/g, bevorzugt 0,001 bis 1 l/g und besonders bevorzugt 0,002 bis 0,2 l/g betragen.

Die Temperatur des Kühlmediums 3 im Sumpf kann im Allgemeinen 27 bis 210 °C, bevorzugt 45 bis 130 °C, insbesondere bevorzugt 55 bis 95 °C betragen. Da die Beladung des Kühlmediums 4 mit Nebenkomponenten im Laufe der Zeit zunimmt, kann ein Teil des beladenen Kühlmediums aus dem Umlauf als Purgestrom 3a abgezogen und die Umlaufmenge durch Zugabe von unbeladenerem Kühlmedium 6 konstant gehalten werden. Das Verhältnis von Ablaufmenge und Zugabemenge hängt von der Dampfbeladung des Produktgases und der Produktgastemperatur am Ende der erste Quenchstufe ab.

Der abgekühlte und eventuell an Nebenkomponenten abgereicherte Produktgasstrom 4 kann nun einer zweiten Quenchstufe C zugeführt werden. In dieser kann er nun erneut mit einem Kühlmedium 9b in Kontakt gebracht werden.

Im Allgemeinen wird das Produktgas bis zum Gasausgang der zweiten Quenchstufe C auf 5 bis 100 °C, vorzugsweise auf 15 bis 85 °C und noch mehr bevorzugt auf 30 bis 70 °C gekühlt. Das Kühlmittel kann im Gegenstrom zum Produktgas zugeführt werden. In diesem Fall kann die Temperatur des Kühlmittelmediums 9b am Kühlmitteleinlass 5 bis 100 °C, bevorzugt 15 bis 85 °C, insbesondere bevorzugt 30 bis 70 °C betragen. Der Druck in der zweiten Quenchstufe C ist nicht besonders eingeschränkt, beträgt aber im Allgemeinen 0,01 bis 4 bar (ü), bevorzugt 0,1 bis 2 bar (ü) und besonders bevorzugt 0,2 bis 1 bar (ü). Die zweite Quenchstufe ist bevorzugt als Kühlturm ausgestaltet. Das im Kühlturm eingesetzte Kühlmedium 9b wird häufig zirkulierend eingesetzt. Der Kreislaufstrom des Kühlmediums 9b in Liter pro Stunde, bezogen auf den Massenstrom an Butadien in Gramm pro Stunde, kann im Allgemeinen 0,0001 bis 5 l/g, bevorzugt 0,001 bis 1 l/g und besonders bevorzugt 0,002 bis 0,2 l/g betragen.

Je nach Temperatur, Druck, Kühlmittel und Wassergehalt des Produktgases 4 kann es in der zweiten Quenchstufe C zur Kondensation von Wasser kommen. In diesem Falle kann sich eine zusätzliche wässrige Phase 8 bilden, welche zusätzlich wasserlösliche Nebenkomponenten enthalten kann. Diese kann dann im Phasenscheider D abgezogen werden. Die Temperatur des Kühlmediums 9 im Sumpf kann im Allgemeinen 20 bis 210 °C, bevorzugt 35 bis 120 °C, insbesondere bevorzugt 45 bis 85 °C betragen. Da die Beladung des Kühlmediums 9 mit Nebenkomponenten im Laufe der Zeit zunimmt, kann ein Teil des beladenen Kühlmediums als Purgestrom 9a aus dem Umlauf abgezogen werden, und die Umlaufmenge durch Zugabe von unbeladenem Kühlmedium 10 konstant gehalten werden.

Um einen möglichst guten Kontakt von Produktgas und Kühlmedium zu erreichen, können Einbauten in der zweiten Quenchstufe C vorhanden sein. Solche Einbauten umfassen zum Beispiel Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak® 250 Y, und Füllkörperkolonnen.

Die Lösungsmittel-Umläufe der beiden Quenchstufen können sowohl voneinander getrennt als auch miteinander verbunden sein. So kann beispielsweise der Strom 9a dem Strom 3b zugeführt werden oder diesen ersetzen. Die gewünschte Temperatur der Umlaufströme kann über geeignete Wärmetauscher eingestellt werden.

In einer bevorzugten Ausführungsform der Erfindung wird also die Abkühlstufe Ca) zweistufig durchgeführt, wobei das mit Nebenkomponenten beladene Lösungsmittel der zweiten Stufe Ca2) in die erste Stufe Ca1) geführt wird. Das der zweiten Stufe Ca2) entnommene Lösungsmittel enthält weniger Nebenkomponenten als das der ersten Stufe Ca1) entnommene Lösungsmittel.

Um den Mitriss von flüssigen Bestandteilen aus dem Quench in die Abgasleitung zu minimieren, können geeignete bauliche Maßnahmen, wie zum Beispiel der Einbau eines Demisters, getroffen werden. Weiterhin können hochsiedende Substanzen, welche im Quench nicht vom Produktgas abgetrennt werdenw, durch weitere bauliche Maßnahmen, wie beispielsweise weitere Gaswäschen, aus dem Produktgas entfernt werden.

Es wird ein Gasstrom 5 erhalten, der n-Butan, 1-Buten, 2-Butene, Butadien, gegebenenfalls Sauerstoff, Wasserstoff, Wasserdampf, in geringen Mengen Methan, Ethan, Ethen, Propan und Propen, iso-Butan, Kohlenstoffoxide, Inertgase und Teile des im Quench verwendeten Lösungsmittels enthält. Weiterhin können in diesem Gasstrom 5 Spuren von hochsiedenden Komponenten verbleiben, welche im Quench nicht quantitativ abgetrennt wurden.

Anschließend wird der Gasstrom b aus dem Abkühlschritt Ca), der an hochsiedenden Nebenkomponenten abgereichert ist, im Schritt Cb) in mindestens einer Kompressionsstufe Cba) und bevorzugt in mindestens einer Abkühlstufe Cbb) durch Inkontaktbringen mit einem organischen Lösungsmittel als Abkühlmittel abgekühlt.

Der Produktgasstrom 5 aus dem Lösungsmittel-Quench wird in mindestens einer Kompressionsstufe E komprimiert und nachfolgend in dem Kühlapparat F weiter abgekühlt, wobei mindestens ein Kondensatstrom 14 entsteht. Es verbleibt ein Gasstrom 12 enthaltend Butadien, 1-Buten, 2-Butene, Sauerstoff, Wasserdampf, gegebenenfalls leichtsiedende Kohlenwasserstoffe wie Methan, Ethan, Ethen, Propan und Propen, Butan und iso-Butan, gegebenenfalls Kohlenstoffoxide und gegebenenfalls Inertgase. Weiterhin kann dieser Produktgasstrom noch Spuren von hochsiedenden Komponenten enthalten.

Die Kompression und Kühlung des Gasstroms 5 kann ein- oder mehrstufig (n-stufig) erfolgen. Im Allgemeinen wird insgesamt von einem Druck im Bereich von 1,0 bis 4,0 bar (absolut) auf einen Druck im Bereich von 3,5 bis 20 bar (absolut) komprimiert. Nach jeder Kompressionsstufe folgt eine Abkühlstufe, in der der Gasstrom auf eine Temperatur im Bereich von 15 bis 60 °C abgekühlt wird. Der Kondensatstrom kann somit bei mehrstufiger Kompression auch mehrere Ströme umfassen. Der Kondensatstrom besteht zu großen Teilen aus Wasser und dem im Quench verwendeten Lösungsmittel. Beide Ströme (wässrige und organische Phase) können daneben in geringem Umfang Nebenkomponenten wie Leichtsieder, C₄-Kohlenwasserstoffe, Oxygenate und Kohlenstoffoxide enthalten.

Um den durch Kompression von Strom 5 entstandenen Strom 11 zu kühlen und/oder um weitere Nebenkomponenten aus dem Strom 11 zu entfernen, kann das kondensierte Quench-Lösungsmittel in einem Wärmetauscher abgekühlt und als Kühlmittel in den Apparat F rückgeführt werden. Da die Beladung dieses Kühlmediums 13b mit Nebenkomponenten im Laufe der Zeit zunimmt, kann ein Teil des beladenen Kühlmediums aus dem Umlauf abgezogen werden (13a) und die Umlaufmenge des Kühlmediums durch Zugabe von unbeladenem Lösungsmittel (15) konstant gehalten werden.

Das Lösungsmittel 15, welches als Kühlmedium zugegeben wird, kann ein wässriges Kühlmittel oder ein organisches Lösungsmittel sein. Bevorzugt sind aromatische Kohlenwasserstoffe, besonders bevorzugt sind Toluol, o-Xylol, m-Xylol, p-Xylol, Diethylbenzol, Trietylbenzol, Diisopropylbenzol, Triisopropylbenzol, Mesitylen oder Gemische daraus. Besonders bevorzugt ist Mesitylen.

Der Kondensatstrom 13a kann in den Kreislaufstrom 3b und/oder 9b des Quenches zurückgeführt werden. Dadurch können die im Kondensatstrom 13a absorbierten C₄-Komponenten wieder in den Gasstrom gebracht und damit die Ausbeute erhöht werden.

Geeignete Verdichter sind beispielsweise Turbo-, Drehkolben- und Hubkolbenverdichter. Die
Verdichter können beispielsweise mit einem Elektromotor, einem Expander oder einer Gas- oder Dampfturbine angetrieben werden. Der Eingangsdruck in die erste Kompressorstufe beträgt 0,5 bis 3 bar absolut, bevorzugt 1 bis 2 bar absolut. Typische Verdichtungsverhältnisse (Austrittsdruck : Eintrittsdruck) pro Verdichterstufe liegen je nach Bauart zwischen 1,5 und 3,0. Die Abkühlung des verdichteten Gases erfolgt in mit Kühlmittel gespülten Wärmetauschern oder organischen Quenchstufen, die beispielsweise als Rohrbündel-, Spiral oder Plattenwärmetauscher ausgeführt sein können. Geeignete Kühlmittel können wässrige oder die oben genannten organischen Lösungsmittel sein. Als Kühlmittel in den Wärmetauschern kommen dabei Kühlwasser oder Wärmeträgeröle oder organische Lösungsmittel zum Einsatz. Daneben wird bevorzugt Luftkühlung unter Einsatz von Gebläsen eingesetzt.

Der Butadien, n-Butene, Sauerstoff, leicht siedende Kohlenwasserstoffe (Methan, Ethan, Ethen, Propan, Propen, n-Butan, iso-Butan), gegebenenfalls Wasserdampf, gegebenenfalls Kohlenstoffoxide sowie gegebenenfalls Inertgase und gegebenenfalls Spuren von Nebenkomponenten enthaltende Gasstrom 12 wird als Ausgangsstrom der weiteren Aufbereitung zugeführt.

In einem Schritt D) werden nicht kondensierbare und leicht siedenden Gasbestandteile, umfassend Sauerstoff, leicht siedenden Kohlenwasserstoffe (Methan, Ethan, Ethen, Propan, Propen), Kohlenstoffoxide und Inertgase in einer Absorptionskolonne G als Gasstrom 16 aus dem Prozessgasstrom 12 durch Absorption der C₄-Kohlenwasserstoffe in einem hochsiedenden Absorptionsmittel (21b und/oder 26) und nachfolgender Desorption der C₄-Kohlenwasserstoffe abgetrennt. Vorzugsweise umfasst der Schritt D), wie in Figur 1 dargestellt, die Schritte Da1), Da2) und Db):
Da1) Absorption der C₄-Kohlenwasserstoffe umfassend Butadien und n-Butene in einem hochsiedenden Absorptionsmittel (21b und/oder 26), wobei ein mit C₄-Kohlenwasserstoffen beladener Absorptionsmittelstrom und der Gasstrom 16 erhalten werden,
Da2) Entfernung von Sauerstoff aus dem mit C₄-Kohlenwasserstoffen beladenen Absorptionsmittelstrom aus Schritt Da1) durch Strippung mit einem nicht kondensierbaren Gasstrom 18, wobei ein mit C₄-Kohlenwasserstoffen beladener Absorptionsmittelstrom 17 erhalten wird, und
Db) Desorption der C₄-Kohlenwasserstoffe aus dem beladenen Absorptionsmittelstrom, wobei ein C₄-Produktgasstrom 27 erhalten wird, der im Wesentlichen aus C₄-Kohlenwasserstoffen besteht.

Dazu wird in der Absorptionsstufe G der Gasstrom 12 mit einem Absorptionsmittel in Kontakt gebracht und werden die C₄-Kohlenwasserstoffe in dem Absorptionsmittel absorbiert, wobei ein mit C₄-Kohlenwasserstoffen beladenes Absorptionsmittel und ein die übrigen Gasbestandteile enthaltendes Abgas 16 erhalten werden. In einer Desorptionsstufe H werden die C₄-Kohlenwasserstoffe aus dem hochsiedenden Absorptionsmittel wieder freigesetzt.

Die Absorptionsstufe kann in jeder beliebigen, dem Fachmann bekannten geeigneten Absorptionskolonne durchgeführt werden. Die Absorption kann durch einfaches Durchleiten des Produktgasstroms durch das Absorptionsmittel erfolgen. Sie kann aber auch in Kolonnen oder in Rotationsabsorbern erfolgen. Dabei kann im Gleichstrom, Gegenstrom oder Kreuzstrom gearbeitet werden. Bevorzugt wird die Absorption im Gegenstrom durchgeführt. Geeignete Absorptionskolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme, Graphitblockabsorber, Oberflächenabsorber wie Dickschicht und Dünnschichtabsorber sowie Rotationskolonnen, Tellerwäscher, Kreuzschleierwäscher und Rotationswäscher in Betracht.

In einer Ausführungsform wird einer Absorptionskolonne im unteren Bereich der Butadien, n-Butene und die leichtsiedenden und nicht kondensierbaren Gasbestandteile enthaltende Gasstrom12 zugeführt. Im oberen Bereich der Absorptionskolonne wird das hochsiedende Absorptionsmittel (21b und/oder 26) aufgegeben.

In der Absorptionsstufe eingesetzte inerte Absorptionsmittel sind im Allgemeinen hochsiedende unpolare Lösungsmittel, in denen das abzutrennende C₄-Kohlenwasserstoff-Gemisch eine deutlich höhere Löslichkeit als die übrigen abzutrennenden Gasbestandteile aufweist. Geeignete Absorptionsmittel sind vergleichsweise unpolare organische Lösungsmittel, beispielsweise aliphatische C₈- bis C₁₈-Alkane, oder aromatische Kohlenwasserstoffe wie die Mittelölfraktionen aus der Paraffindestillation, Toluol oder Ether mit sperrigen Gruppen, oder Gemische dieser Lösungsmittel, wobei diesen ein polares Lösungsmittel wie 1,2-Dimethylphthalat zugesetzt sein kann. Geeignete Absorptionsmittel sind weiterhin Ester der Benzoesäure und Phthalsäure mit geradkettigen C₁- bis C₈-Alkanolen, sowie sogenannte Wärmeträgeröle, wie Biphenyl und Diphenylether, deren Chlorderivate sowie Triarylalkene. Ein geeignetes Absorptionsmittel ist ein Gemisch aus Biphenyl und Diphenylether, bevorzugt in der azeotropen Zusammensetzung, beispielsweise das im Handel erhältliche Diphyl®. Häufig enthält dieses Lösungsmittelgemisch Dimethylphthalat in einer Menge von 0,1 bis 25 Gew.-%.

In einer bevorzugten Ausführungsform in der Absorptionsstufe Da1) wird das gleiche Lösungsmittel wie in der Abkühlstufe Ca) eingesetzt.

Bevorzugte Absorptionsmittel sind Lösungsmittel, die ein Lösungsvermögen für organische Peroxide von mindestens 1000 ppm (mg aktiver Sauerstoff / kg Lösungsmittel) aufweisen. Bevorzugt sind aromatische Kohlenwasserstoffe, besonders bevorzugt Toluol, o-Xylol, p-Xylol und Mesitylen, oder Gemische daraus. Verwendet werden können auch Diethylbenzol, Triethylbenzol, Diisopropylbenzol und Triisopropylbenzol.

Am Kopf der Absorptionskolonne G wird ein Strom 16 abgezogen, der im Wesentlichen Sauerstoff, leicht siedende Kohlenwasserstoffe (Methan, Ethan, Ethen, Propan, Propen), gegebenenfalls C₄-Kohlenwasserstoffe (Butan, Butene, Butadien), gegebenenfalls Inertgase, gegebenenfalls Kohlenstoffoxide und gegebenenfalls noch Wasserdampf enthält. Dieser Stoffstrom kann teilweise dem ODH-Reaktor zugeführt werden. Damit lässt sich zum Beispiel der Eintrittsstrom des ODH-Reaktors auf den gewünschten C₄-Kohlenwasserstoffgehalt einstellen.

Am Sumpf der Absorptionskolonne werden durch die Spülung mit einem Gas 18 Reste von im Absorptionsmittel gelöstem Sauerstoff ausgetragen. Der verbleibende Sauerstoffanteil soll so klein sein, dass der die Desorptionskolonne verlassende und Butan, Buten sowie Butadien enthaltende Strom 27 nur noch maximal 100 ppm Sauerstoff enthält.

Das Ausstrippen des Sauerstoffs in Schritt Db) kann in jeder beliebigen, dem Fachmann bekannten geeigneten Kolonne durchgeführt werden. Das Strippen kann durch einfaches Durchleiten von nicht kondensierbaren Gasen, vorzugsweise nicht oder nur schwach im Absorptionsmittelstrom 21b und/oder 26 absorbierbare Gase wie Methan, durch die beladene Absorptionslösung erfolgen. Mit ausgestrippte C₄-Kohlenwasserstoffe werden im oberen Teil der Kolonne G zurück in die Absorptionslösung gewaschen, indem der Gasstrom in diese Absorptionskolonne zurück geleitet wird. Das kann sowohl durch eine Verrohrung der Stripperkolonne als auch eine direkte Montage der Stripperkolonne unterhalb der Absorberkolonne erfolgen. Da der Druck im Strippkolonnenteil und Absorptionskolonnenteil gleich ist, kann diese direkte Kopplung erfolgen. Geeignete Stippkolonnen sind z. B. Bodenkolonnen mit Glocken-, Zentrifugal- und/oder Siebboden, Kolonnen mit strukturierten Packungen, z. B. Blechpackungen mit einer spezifischen Oberfläche von 100 bis 1000 m²/m³ wie Mellapak® 250 Y, und Füllkörperkolonnen. Es kommen aber auch Riesel- und Sprühtürme sowie Rotationskolonnen, Tellerwäscher, Kreuzschleierwäscher und Rotationswäscher in Betracht. Geeignete Gase sind zum Beispiel Stickstoff oder Methan.

Der Strom 17 kann gegebenenfalls abgekühlt oder erhitzt werden und tritt als Strom 19 in die Desorptionskolonne H ein. Die Eintrittsstelle liegt im Allgemeinen 0 bis 10 theoretische Trennstufen, bevorzugt 2 bis 8 theoretische Trennstufen, besonders bevorzugt 3 bis 5 theoretische Trennstufen unterhalb des Kolonnenkopfes.

Das in der Desorptionsstufe regenerierte Absorptionsmittel wird als Strom 20 zusammen mit dem kondensierten Wasser aus der Desorptionskolonne H entnommen. Dieses zweiphasige Gemisch kann in einem Wärmetauscher abgekühlt werden und als Strom 21 in einem Dekanter I in einen wässrigen Strom 21a und einen Absorbensstrom 21b getrennt werden. Der Absorbensstrom 21b wird wieder der Absorberkolonne G zugeführt werden, während der wässrige Strom 21a in einem Verdampfer verdampft wird und damit der Strom 23 erzeugt wird. Zusätzlich oder alternativ kann noch zusätzliches Wasser (Strom 24) im Verdampfer verdampft werden. Weiter kann auch nur ein Teil des Stroms 21a verdampft werden und der unverdampfte Teil als Strom 22 entnommen und zum Beispiel der Abwasserbehandlung zugeführt werden.

Im Prozessgasstrom befindliche Leichtsieder wie beispielsweise Ethan oder Propan sowie schwersiedende Komponenten wie Benzaldehyd, Maleinsäureanhydrid und Phthalsäureanhydrid können sich im Absorptionsmittelkreislaufstrom anreichern. Um die Anreicherung zu begrenzen, kann ein Purgestrom 25 abgezogen werden.

Der im Wesentlichen aus n-Butan, n-Butenen und Butadien bestehende C₄-Produktgasstrom 27 enthält im Allgemeinen 20 bis 80 Vol.-% Butadien, 0 bis 80 Vol.-% n-Butan, 0 bis 10 Vol.-% 1-Buten und 0 bis 50 Vol.-% 2-Butene, wobei die Gesamtmenge 100 Vol.-% ergibt. Weiterhin können geringe Mengen an iso-Butan enthalten sein.

Ein Teil des kondensierten, hauptsächlich C₄-Kohlenwasserstoffe enthaltenen Kopfaustrags der Desorptionskolonne wird als Strom 30 in den Kolonnenkopf zurückgeführt, um die Trennleistung der Kolonne zu erhöhen.

Die Desorptionsstufe H kann in jeder beliebigen, dem Fachmann bekannten geeigneten Desorptionskolonne durchgeführt werden. Die Desorption kann durch Absenken des Drucken und/oder Erhitzen der Desorptionsstufe erfolgen. Die Desorptionsstufe kann durch Zuführen eines heißen Mediums - wie zum Beispiel Wasserdampf - oder durch Eigendampf, der z.B. durch Teilverdampfen der Absorptionslösung im Sumpf der Desorberkolonne erzeugt wird, erhitzt werden.

Geeignete Desorptionskolonnen sind z. B. Bodenkolonnen mit Glocken-, Zentrifugal- und/oder Siebböden, Kolonnen mit strukturierten Packungen, z.B. Blechpackungen mit einer spezifischen Oberfläche von 100 bis 1000 m²/m³ wie Mellapak® 250 Y, und Füllkörperkolonnen. Erfindungsgemäß wird, wie in Figur 1 gezeigt, der Desorptionskolonne H ein Methacrolein enthaltender Seitenabzugsstrom 31 entnommen, um die Aufpegelung von Acrolein im Absorptionsmittel-Kreislaufstrom zu verhindern. Der Seitenabzugsstrom 31 kann sowohl flüssig als auch gasförmig sein, bevorzugt ist er gasförmig.

Bevorzugt weist die Desorptionskolonne H 5 bis 30, besonders bevorzugt 10 bis 20 theoretische Böden auf. Der Seitenabzugsstrom 31 wird dabei bevorzugt im unteren Drittel der Desorptionskolonne entnommen. Der flüssige Seitenabzugsstrom 31 enthält im Allgemeinen 0,1 bis 2 Gew.-% Methacrolein. Daneben enthält er 5 bis 15 Gew.-% Wasser, 0 bis 3 Gew.-% C₄-Kohlenwasserstoffe und 70 bis 90 Gew.-% des Absorptionsmittels.

Der gasförmige Seitenabzugsstrom 31 enthält im Allgemeinen 1 bis 10 Gew.-% Methacrolein. Daneben enthält er 30 bis 60 Gew.-% Wasser, 0 bis 6 Gew.-% C₄-Kohlenwasserstoffe und 30 bis 60 Gew.-% des Absorptionsmittels.

Der gasförmige, C₄-Kohlenwasserstoffe enthaltende Strom 29 wird zum Kompressor E zurückgeführt.

Vorzugsweise wird dem Kopfkondensator der Desorptionskolonne H der Polymerisationsinhibitor mit dem Strom 27a zugesetzt. Dieser kann in fester Form, als Lösung oder Emulsion zugegeben werden. Bevorzugt wird er als Lösung zuzugeben. Besonders bevorzugt wird er als wässrige Lösung zugegeben. Die Lösung kann einen oder mehrere verschiedene Stabilisatoren enthalten. Bevorzugte Polymerisationsinhibitoren (Stabilisatoren) sind ausgewählt aus der Gruppe der unsubstituierten und substituierten Brenzcatechine und Hydrochinone.

Im Allgemeinen wird der Polymerisationsinhibitor in solchen Mengen zugegeben, dass seine Konzentration dem am Kopfkondensator erhaltenen flüssigen Kondensat von 10 bis 500 ppm, bevorzugt 30 bis 100 ppm beträgt.

Somit beträgt die Konzentration des Polymerisationsinhibitors im Strom 28 und im Rücklauf 30 10 bis 500 ppm, vorzugsweise 30 bis 100 ppm.

Bevorzugt ist ein Gemisch von mindestens einem Stabilisator aus der Klasse der Brenzcatechine und mindestens einem Stabilisator aus der Klasse der Hydrochinone. Besonders bevorzugt ist ein Gemisch aus tert.-Butylbrenzcatechin und 4-Methoxyphenol.

Der den Kopfkondensator verlassende, flüssige Strom 28 enthaltend die C₄-Kohlenwasserstoffe wird anschließend in Stufe N verdampft und der resultierende Strom 28a durch Extraktivdestillation im Schritt E) mit einem für Butadien selektiven Lösungsmittel in einen Butadien und das selektive Lösungsmittel enthaltenden Stoffstrom 35 und einen Butane und n-Butene enthaltenden Stoffstrom 36 aufgetrennt.

In einer Ausführung kann der Strom 28 zuvor in einer Flüssig-Flüssig-Wäsche mit Polyalkoholen wie Ethylenglykol und Glycerin oder Methanol gewaschen werden und so darin enthaltenes Furan teilweise abgetrennt werden. In einer weiteren Ausführung kann der Strom 28a zuvor in einer Gas-Flüssig-Wäsche mit Wasser von anderen Nebenkomponenten wie Aldehyden befreit werden.

Die Extraktivdestillation kann beispielsweise, wie in "Erdöl und Kohle - Erdgas - Petrochemie", Band 34 (8), Seiten 343 bis 346 oder "Ullmanns Enzyklopädie der Technischen Chemie", Band 9, 4. Auflage 1975, Seiten 1 bis 18 beschrieben, durchgeführt werden. Hierzu wird der C₄-Produktgasstrom mit einem Extraktionsmittel, vorzugsweise einem N-Methylpyrrolidon (NMP)/Wasser-Gemisch, in einer Extraktionszone in Kontakt gebracht. Die Extraktionszone ist im Allgemeinen in Form einer Waschkolonne ausgeführt, welche Böden, Füllkörper oder Packungen als Einbauten enthält. Diese weist im Allgemeinen 30 bis 70 theoretische Trennstufen auf, damit eine hinreichend gute Trennwirkung erzielt wird. Vorzugsweise weist die Waschkolonne im Kolonnenkopf eine Rückwaschzone auf. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Das Massenverhältnis Extraktionsmittel zu C₄-Produktgasstrom im Zulauf der Extraktionszone beträgt im Allgemeinen 10 : 1 bis 20 : 1. Die Extraktivdestillation wird vorzugsweise bei einer Sumpftemperatur im Bereich von 100 bis 250 °C, insbesondere bei einer Temperatur im Bereich von 110 bis 210 °C, einer Kopftemperatur im Bereich von 10 bis 100·°C, insbesondere im Bereich von 20 bis 70·°C und einem Druck im Bereich von 1 bis 15 bar, insbesondere im Bereich von 3 bis 8 bar betrieben. Die Extraktivdestillationskolonne weist vorzugsweise 5 bis 70 theoretische Trennstufen auf.

Geeignete Extraktionsmittel sind Butyrolacton, Nitrile wie Acetonitril, Propionitril, Methoxypropionitril, Ketone wie Aceton, Furfural, N-alkylsubstituierte niedere aliphatische Säureamide wie Dimethylformamid, Diethylformamid, Dimethylacetamid, Diethylacetamid, N-Formylmorpholin, N-alkylsubstituierte zyklische Säureamide (Lactame) wie N-Alkylpyrrolidone, insbesondere N-Methylpyrrolidon (NMP). Im Allgemeinen werden alkylsubstituierte niedere aliphatische Säureamide oder N-alkylsubstituierte zyklische Säureamide verwendet. Besonders vorteilhaft sind Dimethylformamid, Acetonitril, Furfural und insbesondere NMP.

Es können jedoch auch Mischungen dieser Extraktionsmittel untereinander, z.B. von NMP und Acetonitril, Mischungen dieser Extraktionsmittel mit Co-Lösungsmitteln und/oder tert.-Butylether, z.B. Methyl-tert.-butylether, Ethyl-tert.-butylether, Propyl-tert.-butylether, n- oder iso-Butyl-tert.-butylether eingesetzt werden. Besonders geeignet ist NMP, bevorzugt in wässriger Lösung, vorzugsweise mit 0 bis 20 Gew.-% Wasser, besonders bevorzugt mit 7 bis 10 Gew.-% Wasser, insbesondere mit 8,3 Gew.-% Wasser.

Der Kopfproduktstrom 36 der Extraktivdestillationskolonne J enthält im Wesentlichen Butan und Butene und in geringen Mengen Butadien und wird gasförmig oder flüssig abgezogen. Im Allgemeinen enthält der im Wesentlichen aus n-Butan und 2-Buten bestehende Strom bis 100 Vol.-% n-Butan, 0 bis 50 Vol.-% 2-Buten und 0 bis 3 Vol.-% weitere Bestandteile wie Isobutan, Isobuten, Propan, Propen und C₅⁺-Kohlenwasserstoffe.

Der im Wesentlichen aus n-Butan und 2-Buten und gegebenenfalls Methan bestehende Strom kann ganz oder teilweise oder auch nicht dem C₄-Feed des ODH-Reaktors zugeführt werden. Da die Buten-Isomere dieses Rückführstroms im Wesentlichen aus 2-Butenen bestehen, und 2-Butene im Allgemeinen langsamer zu Butadien oxidativ dehydriert werden als 1-Buten, kann dieser Rückführstrom vor der Zuführung in den ODH-Reaktor katalytisch isomerisiert werden. Dadurch kann die Isomerenverteilung entsprechend der im thermodynamischen Gleichgewicht vorliegenden Isomerenverteilung eingestellt werden. Weiterhin kann der Strom auch einer weiteren Aufarbeitung zugeführt werden, um Butane und Butene voneinander zu trennen und die Butene ganz oder teilweise in die Oxidehydrierung zurückzuführen. Der Strom kann auch in die Maleinsäureanhydrid-Produktion gehen.

In einem Schritt F) wird der Butadien und das selektive Lösungsmittel enthaltende Stoffstrom in einen im Wesentlichen aus dem selektiven Lösungsmittel bestehenden Stoffstrom und einen Butadien enthaltenden Stoffstrom destillativ aufgetrennt.

Der am Sumpf der Extraktivdestillationskolonne J gewonnene Stoffstrom 35 enthält im Allgemeinen das Extraktionsmittel, Wasser, Butadien und in geringen Anteilen Butene und Butan und wird einer Destillationskolonne K zugeführt. In dieser kann über Kopf oder als Seitenabzug Butadien gewonnen werden. Am Sumpf der Destillationskolonne fällt ein Extraktionsmittel und gegebenenfalls Wasser enthaltender Stoffstrom 37 an, wobei die Zusammensetzung des Extraktionsmittel und Wasser enthaltenden Stoffstroms der Zusammensetzung entspricht, wie sie der Extraktion zugegeben wird. Der Extraktionsmittel und Wasser enthaltende Stoffstrom wird bevorzugt in die Extraktivdestillation zurückgeleitet.

Falls das Butadien über einen Seitenabzug gewonnen wird, wird die so abgezogene Extraktionslösung in eine Desorptionszone überführt, wobei aus der Extraktionslösung das Butadien nochmals desorbiert und rückgewaschen wird. Die Desorptionszone kann beispielsweise in Form einer Waschkolonne ausgeführt sein, die 2 bis 30, bevorzugt 5 bis 20 theoretische Stufen und gegebenenfalls eine Rückwaschzone mit beispielsweise 4 theoretischen Stufen aufweist. Diese Rückwaschzone dient zur Rückgewinnung des in der Gasphase enthaltenen Extraktionsmittels mit Hilfe eines flüssigen Kohlenwasserstoffrücklaufs, wozu die Kopffraktion zuvor kondensiert wird. Als Einbauten sind Packungen, Böden oder Füllkörper vorgesehen. Die Destillation wird vorzugsweise bei einer Sumpftemperatur im Bereich von 100 bis 300 °C, insbesondere im Bereich von 150 bis 200 °C und einer Kopftemperatur im Bereich von 0 bis 70 °C, insbesondere im Bereich von 10 bis 50 °C durchgeführt. Der Druck in der Destillationskolonne liegt dabei vorzugsweise im Bereich von 1 bis 10 bar. Im Allgemeinen herrschen in der Desorptionszone gegenüber der Extraktionszone ein verminderter Druck und/oder eine erhöhte Temperatur.

Der am Kolonnenkopf gewonnene Wertproduktstrom 38 enthält im Allgemeinen 90 bis 100 Vol.-% Butadien, 0 bis 10 Vol.-% 2-Buten und 0 bis 10 Vol.-% n-Butan und iso-Butan. Zur weiteren Aufreinigung des Butadiens kann eine weitere Destillation nach dem Stand der Technik durchgeführt werden.

Die beanspruchte technische Lösung wurde über thermodynamische Gleichgewichtsstufensimulationen entwickelt und in einer Pilotanlage überprüft.
Diese Pilotanlage umfasst den Salzbadreaktor, den organischen Quench, die Kompressoreinheit sowie die C₄-Absorption/Desorption-Einheit. Der Maßstab der Pilotanlage ist so gewählt worden, dass ein Up-Scaling auf eine Großanlage möglich ist. Dementsprechend repräsentativ wurden zum Beispiel die Einbauten der Kolonnen und die Rückführungen gewählt. Die Pilotanlage kann zwischen 500 und 1500 Gramm Butadien pro Stunde herstellen.

### Vergleichsbeispiel:

Beim Betrieb der Anlage wurden nach 10 Tagen Betrieb polymere Ablagerungen aus einem Metacrolein-Butadien-Copolymer im obersten Kolonnenschuss der Desorberkolonne H gefunden.

Weiterhin wurden auch im C4-Verdampfer (N in Figur 1) polymere Ablagerungen gefunden. Die Polymerbildung war an dieser Stelle so dominant, dass der Rohrwendelverdampfer der Anlage verstopfte.

### Beispiel 1:

Die gefundenen Polymere sind mit hoher Wahrscheinlichkeit über einen radikalischen Mechanismus gebildet worden. Als Gegenmaßnahme wurde dem Kondensator am Kopf der Desorberkolonne ein Radikalfänger zugegeben. Durch Wahl dieser Zugabestelle wurden sowohl der Kondensator als auch nachfolgende Bauteile durch den Radikalfänger vor Polymeren geschützt. Dieser verteilte sich über den C4-Rücklaufstrom 30 in die Desorberkolonne und schützt die oberen Kolonnenschüsse, die durch eine Inhibierung des im Kreislauf geführten Absorbens nicht geschützt werden. Weiter fließt er auch mit dem Strom 28 in den C4-Verdampfer und unterbindet auch dort die Bildung von Polymeren. In Experimenten in der Miniplant-Anlage mit den nachstehend tabellierten Flüssen wurde gezeigt, dass durch die Maßnahmen ein störungsfreier Betrieb erreicht wurde.

Die Zusammensetzung der einzelnen Stoffströme ist in Tabelle 1 wiedergegeben.

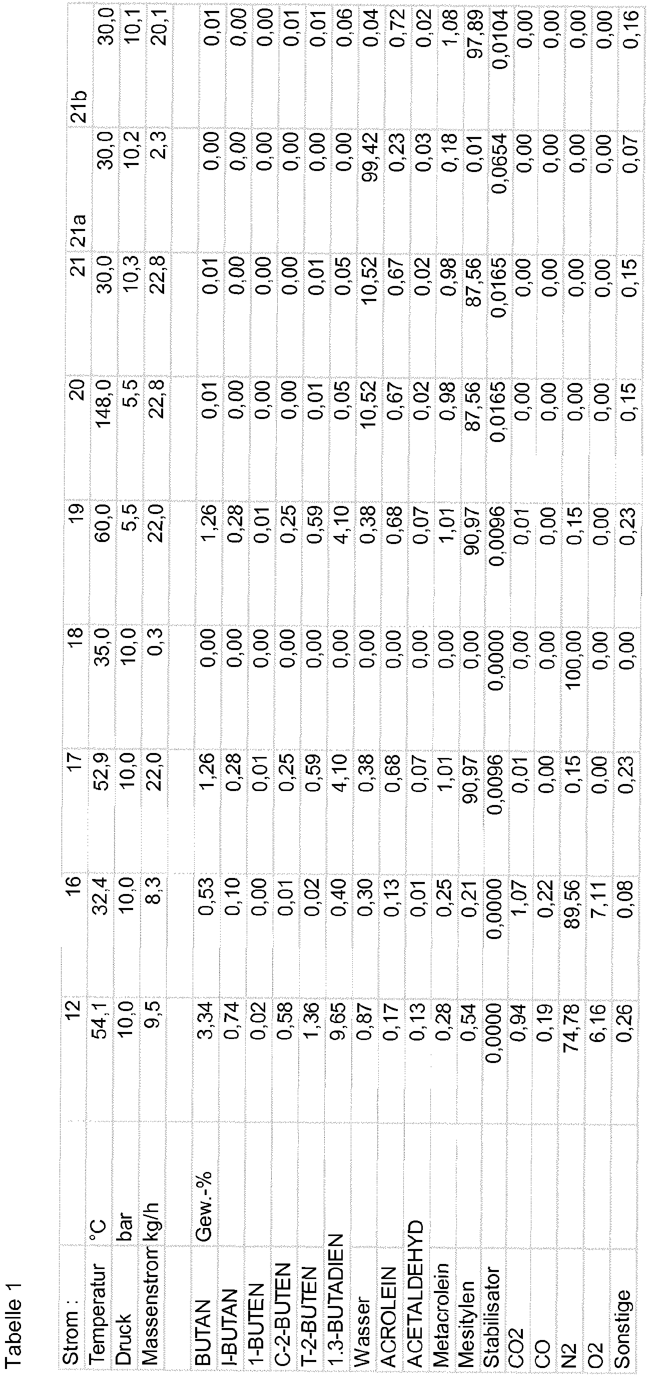

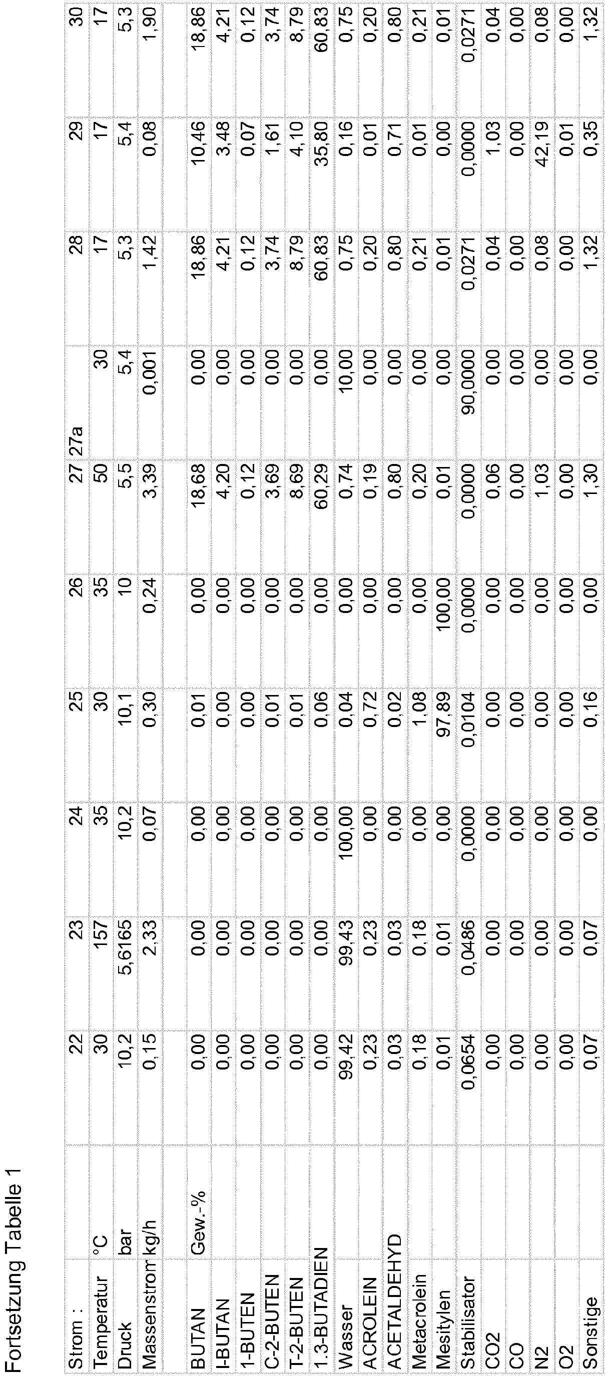

## Patentansprüche

1. Verfahren zur Herstellung von Butadien aus n-Butenen mit den Schritten:
A) Bereitstellung eines n-Butene enthaltenden Einsatzgasstroms a;
B) Einspeisung des n-Butene enthaltenden Einsatzgasstromes a und eines sauerstoffhaltigen Gases in mindestens eine oxidative Dehydrierzone und oxidative Dehydrierung von n-Butenen zu Butadien, wobei ein Produktgasstrom b enthaltend Butadien, nicht umgesetzte n-Butene, Wasserdampf, Sauerstoff, leicht siedende Kohlenwasserstoffe, hochsiedende Nebenkomponenten, gegebenenfalls Kohlenstoffoxide und gegebenenfalls Inertgase erhalten wird;
Ca) Abkühlung des Produktgasstroms b durch Inkontaktbringen mit einem Kühlmittel und Kondensation zumindest eines Teils der hochsiedenden Nebenkomponenten;
Cb) Kompression des verbleibenden Produktgasstroms b in mindestens einer Kompressionsstufe, wobei mindestens ein wässriger Kondensatstrom c1 und ein Gasstrom c2 enthaltend Butadien, n-Butene, Wasserdampf, Sauerstoff, leicht siedende Kohlenwasserstoffe, gegebenenfalls Kohlenstoffoxide und gegebenenfalls Inertgase erhalten wird;
Da) Abtrennung von nicht kondensierbaren und leicht siedenden Gasbestandteilen umfassend Sauerstoff, leicht siedende Kohlenwasserstoffe, gegebenenfalls Kohlenstoffoxide und gegebenenfalls Inertgase als Gasstrom d2 aus dem Gasstrom c2 durch Absorption der C₄-Kohlenwasserstoffe umfassend Butadien und n-Butene in einem Absorptionsmittel, wobei ein mit C₄-Kohlenwasserstoffen beladener Absorptionsmittelstrom und der Gasstrom d2 erhalten werden, und
Db) anschließende Desorption der C₄-Kohlenwasserstoffe aus dem beladenen Absorptionsmittelstrom in einer Desorptionskolonne, wobei ein C₄-Produktgasstrom d1 erhalten wird,
**dadurch gekennzeichnet, dass** in Schritt Db) am Kolonnenkopf der Desorptionskolonne ein Polymerisationsinhibitor zugegeben wird.

2. Verfahren nach Anspruch 1 mit den zusätzlichen Schritten:
E) Auftrennung des C₄-Produktstroms d1 durch Extraktivdestillation mit einem für Butadien selektiven Lösungsmittel in einen Butadien und das selektive Lösungsmittel enthaltenden Stoffstrom e1 und einen n-Butene enthaltenden Stoffstrom e2;
F) Destillation des Butadien und das selektive Lösungsmittel enthaltenden Stoffstroms e1 in einen im Wesentlichen aus dem selektiven Lösungsmittel bestehenden Stoffstrom f1 und einen Butadien enthaltenden Stoffstrom f2.

3. Verfahren nach Anspruche 1 oder 2, **dadurch gekennzeichnet, dass** der Polymerisationsinhibitor am Kopfkondensator der Desorptionskolonne zugegeben wird.

4. Verfahren nach Anspruch 3, **dadurch gekennzeichnet, dass** der Polymerisationsinhibitor in solchen Mengen zugegeben wird, dass die Konzentration des Polymerisationsinhibitors in dem am Kopfkondensator erhaltenen flüssigen Kondensatstrom von 10 bis 1500 ppm beträgt.

5. Verfahren nach einem der Ansprüche 1 bis 4, **dadurch gekennzeichnet, dass** der Poly-merisationsinhibitor ausgewählt ist aus der Gruppe bestehend aus unsubstituierten oder substituierten Brenzcatechinen oder Hydrochinonen.

6. Verfahren nach Anspruch 4 oder 5, **dadurch gekennzeichnet, dass** der Polymerisationsin-hibitor ein Gemisch ist aus tert.-Butylbrenzcatechin und 4-Methoxyphenol.

7. Verfahren nach einem der Ansprüche 1 bis 6, **dadurch gekennzeichnet, dass** das in Schritt B) eingespeiste sauerstoffhaltige Gas mindestens 90 Vol.-% Sauerstoff enthält.

8. Verfahren nach einem der Ansprüche 1 bis 7, **dadurch gekennzeichnet, dass** der in Schritt Da) abgetrennte Gasstrom d2 zumindest teilweise in Schritt B) zurückgeführt wird.

9. Verfahren nach einem der Ansprüche 1 bis 8, **dadurch gekennzeichnet**, das Schritt Da) die Schritte Da1) und Da2) umfasst:
Da1) Absorption der C₄-Kohlenwasserstoffe umfassend Butadien und n-Butene in einem hochsiedenden Absorptionsmittel, wobei ein mit C₄-Kohlenwasserstoffen beladener Absorptionsmittelstrom und der Gasstrom d2 erhalten werden,
Da2) Entfernung von Sauerstoff aus dem mit C₄-Kohlenwasserstoffen beladenen Absorptionsmittelstrom aus Schritt Da) durch Strippung mit einem nicht kondensierbaren Gasstrom, und
Db) Desorption der C₄-Kohlenwasserstoffe aus dem beladenen Absorptionsmittelstrom, wobei ein C₄-Produktgasstrom d1 erhalten wird, der weniger als 100 ppm Sauerstoff umfasst.

10. Verfahren nach einem der Ansprüche 1 bis 9, **dadurch gekennzeichnet, dass** das in Schritt Da) eingesetzte Absorptionsmittel ein aromatisches Kohlenwasserstofflösungsmittel ist.

## Claims

1. A method for producing butadiene from n-butenes having the steps:
A) providing a feed gas stream a comprising n-butenes;
B) feeding the feed gas stream a comprising n-butenes and an oxygen-comprising gas into at least one oxidative dehydrogenation zone and oxidatively dehydrogenating n-butenes to butadiene, wherein a product gas stream b comprising butadiene, unreacted n-butenes, steam, oxygen, low-boiling hydrocarbons, high-boiling minor components, possibly carbon oxides and possibly inert gases is obtained;
Ca) cooling the product gas stream b by contacting it with a refrigerant and condensing at least a part of the high-boiling minor components;
Cb) compressing the remaining product gas stream b in at least one compression stage, wherein at least one aqueous condensate stream c1 and a gas stream c2 comprising butadiene, n-butenes, steam, oxygen, low-boiling hydrocarbons, possibly carbon oxides and possibly inert gases are obtained;
Da) separating off non-condensable and low-boiling gas components comprising oxygen, low-boiling hydrocarbons, possibly carbon oxides and possibly inert gases as gas stream d2 from the gas stream c2 by absorbing the C₄ hydrocarbon-comprising butadiene and n-butenes in an absorbent, wherein an absorbent stream loaded with C₄ hydrocarbons and the gas stream d2 are obtained, and
Db) subsequent desorption of the C₄ hydrocarbons from the loaded absorbent stream in a desorption column, wherein a C₄ product gas stream d1 is obtained,
wherein a polymerization inhibitor is added in step Db) at the column head of the desorption column.

2. The method according to claim 1 having the additional steps:
E) separating the C₄ product stream d1 by extractive distillation using a solvent selective for butadiene into a material stream e1 comprising butadiene and the selective solvent, and a material stream e2 comprising n-butenes;
F) distilling the material stream e1 comprising butadiene and the selective solvent into a material stream f1 substantially comprising the selective solvent, and a material stream f2 comprising butadiene.

3. The method according to claims 1 or 2, wherein the polymerization inhibitor is added at the top condenser of the desorption column.

4. The method according to claim 3, wherein the polymerization inhibitor is added in amounts such that the concentration of the polymerization inhibitor in the liquid condensate stream obtained at the top condenser is from 10 to 1500 ppm.

5. The method according to any one of claims 1 to 4, wherein the polymerization inhibitor is selected from the group consisting of unsubstituted or substituted catechols or hydroquinones.

6. The method according to claim 4 or 5, wherein the polymerization inhibitor is a mixture of tert-butyl catechol and 4-methoxyphenol.

7. The method according to any one of claims 1 to 6, wherein the oxygen-comprising gas fed in in step B) comprises at least 90% by volume of oxygen.

8. The method according to any one of claims 1 to 7, wherein the gas stream d2 that is separated off in step Da) is at least in part recirculated in step B) .

9. The method according to any one of claims 1 to 8, wherein the step Da) comprises the steps Da1) and Da2) :
Da1) absorption of the C₄ hydrocarbons comprising butadiene and n-butenes in a high-boiling absorbent, wherein an absorbent stream loaded with C₄ hydrocarbons and the gas stream d2 are obtained,
Da2) removal of oxygen from the absorbent stream of step Da) that is loaded with C₄ hydrocarbons by stripping with a non-condensable gas stream, and
Db) desorption of the C₄ hydrocarbons from the loaded absorbent stream, wherein a C₄- product gas stream d1 is obtained which comprises less than 100 ppm of oxygen.

10. The method according to any one of claims 1 to 9, wherein the absorbent used in step Da) is an aromatic hydrocarbon solvent.

## Revendications

1. Procédé de fabrication de butadiène à partir de n-butènes comprenant les étapes suivantes :
A) la préparation d'un courant gazeux d'entrée contenant des n-butènes a ;
B) l'introduction du courant gazeux d'entrée contenant des n-butènes a et d'un gaz contenant de l'oxygène dans au moins une zone de déshydrogénation oxydative et la déshydrogénation oxydative de n-butènes en butadiène, un courant gazeux de produits b contenant du butadiène, des n-butènes non réagis, de la vapeur d'eau, de l'oxygène, des hydrocarbures de point d'ébullition faible, des composants secondaires de point d'ébullition élevé, éventuellement des oxydes de carbone et éventuellement des gaz inertes étant obtenu ;
Ca) le refroidissement du courant gazeux de produits b par mise en contact avec un agent réfrigérant et condensation d'au moins une partie des composants secondaires de point d'ébullition élevé ;
Cb) la compression du courant gazeux de produits b restant dans au moins une étape de compression, au moins un courant de condensat aqueux c1 et un courant gazeux c2 contenant du butadiène, des n-butènes, de la vapeur d'eau, de l'oxygène, des hydrocarbures de point d'ébullition faible, éventuellement des oxydes de carbone et éventuellement des gaz inertes étant obtenus ;
Da) la séparation de constituants gazeux non condensables et de point d'ébullition faible comprenant de l'oxygène, des hydrocarbures de point d'ébullition faible, éventuellement des oxydes de carbone et éventuellement des gaz inertes en tant que courant gazeux d2 à partir du courant gazeux c2 par absorption des hydrocarbures en C₄ comprenant du butadiène et des n-butènes dans un agent d'absorption, un courant d'agent d'absorption chargé avec des hydrocarbures en C₄ et le courant gazeux d2 étant obtenus, puis
Db) la désorption des hydrocarbures en C₄ du courant d'agent d'absorption chargé dans une colonne de désorption, un courant gazeux de produits en C₄ d1 étant obtenu,
**caractérisé en ce qu'**à l'étape Db), un inhibiteur de polymérisation est ajouté à la tête de colonne de la colonne de désorption.

2. Procédé selon la revendication 1, comprenant les étapes supplémentaires suivantes :
E) la séparation du courant de produits en C₄ d1 par distillation extractive avec un solvant sélectif pour le butadiène en un courant de matières e1 contenant du butadiène et le solvant sélectif, et un courant de matières e2 contenant des n-butènes ;
F) la distillation du courant de matières e1 contenant du butadiène et le solvant sélectif en un courant de matières f1 essentiellement constitué par le solvant sélectif et un courant de matières f2 contenant du butadiène.

3. Procédé selon la revendication 1 ou 2, **caractérisé en ce que** l'inhibiteur de polymérisation est ajouté dans le condensateur de tête de la colonne de désorption.

4. Procédé selon la revendication 3, **caractérisé en ce que** l'inhibiteur de polymérisation est ajouté en quantités telles que la concentration de l'inhibiteur de polymérisation dans le courant de condensat liquide obtenu dans le condensateur de tête soit de 10 à 1 500 ppm.

5. Procédé selon l'une quelconque des revendications 1 à 4, **caractérisé en ce que** l'inhibiteur de polymérisation est choisi dans le groupe constitué par les brenzcatéchines ou les hydroquinones non substituées ou substituées.

6. Procédé selon la revendication 4 ou 5, **caractérisé en ce que** l'inhibiteur de polymérisation est un mélange de tert.-butylbrenzcatéchine et de 4-méthoxyphénol.

7. Procédé selon l'une quelconque des revendications 1 à 6, **caractérisé en ce que** le gaz contenant de l'oxygène introduit à l'étape B) contient au moins 90 % en volume d'oxygène.

8. Procédé selon l'une quelconque des revendications 1 à 7, **caractérisé en ce que** le courant gazeux d2 séparé à l'étape Da) est au moins partiellement recyclé dans l'étape B).

9. Procédé selon l'une quelconque des revendications 1 à 8, **caractérisé en ce que** l'étape Da) comprend les étapes Da1) et Da2):
Da1) l'absorption des hydrocarbures en C₄ comprenant du butadiène et des n-butènes dans un agent d'absorption de point d'ébullition élevé, un courant d'agent d'absorption chargé avec des hydrocarbures en C₄ et le courant gazeux d2 étant obtenus,
Da2) l'élimination d'oxygène à partir du courant d'agent d'absorption chargé avec des hydrocarbures en C₄ de l'étape Da) par extraction avec un courant gazeux non condensable, et
Db) la désorption des hydrocarbures en C₄ à partir du courant d'agent d'absorption chargé, un courant gazeux de produits en C₄ d1 étant obtenu, qui comprend moins de 100 ppm d'oxygène.

10. Procédé selon l'une quelconque des revendications 1 à 9, **caractérisé en ce que** l'agent d'absorption utilisé à l'étape Da) est un solvant hydrocarboné aromatique.
